# EUROPÄISCHE PATENTSCHRIFT

(11) **EP 0 788 475 B1**
(45) Veröffentlichungstag und Bekanntmachung des Hinweises auf die Patenterteilung: **20.10.1999**
(21) Anmeldenummer: 95917966.4
(22) Anmeldetag: 26.04.1995
(51) Int. Cl.: C07C 239/20, C07C 309/66, C07C 249/12

(54) **VERFAHREN ZUR HERSTELLUNG VON ISOMERENGEMISCHEN AUS O-PHENOXYALKYLHYDROXYLAMINEN ODER O-PHENOXYALKYLOXIMEN**
PROCESS FOR PRODUCING ISOMER MIXTURES FROM O-PHENOXYALKYLHYDROXYL AMINES OR O-PHENOXYALKYLOXIMES
PROCEDE DE PRODUCTION DE MELANGES ISOMERES DE O-PHENOXYALKYLHYDROXYLAMINES OU DE O-PHENOXYALKYLOXIMES

(30) Priorität: 05.05.1994 DE 4415887
(43) Veröffentlichungstag der Anmeldung: 13.08.1997
(73) Patentinhaber: BASF AKTIENGESELLSCHAFT, 67056 Ludwigshafen (DE)
(72) Erfinder: RANG, Harald, D-67122 Altrip (DE); GÖTZ, Norbert, D-67547 Worms (DE); HARREUS, Albrecht, D-67063 Ludwigshafen (DE); BORCHERS, Dirk, D-67134 Birkenheide (DE); HARTMANN, Horst, 67459 Böhl-Iggelheim (DE); MAYWALD, Volker, D-67069 Ludwigshafen (DE); HEIMANN, Frank, D-67065 Ludwigshafen (DE); BUSCHULTE, Thomas, D-67459 Böhl-Iggelheim (DE)
(86) Internationale Anmeldenummer: EP9501581
(87) Internationale Veröffentlichungsnummer: WO9530648

(56) Entgegenhaltungen:
- DE-A- 4 204 203
- GB-A- 2 115 812

## Beschreibung

Die vorliegende Erfindung betrifft ein Verfahren zur Herstellung von Isomerengemischen aus O-Phenoxyalkylhydroxylaminen der allgemeinen Formeln Ia und Ib

H₂N-O-CH₂-CH(R¹)-O-Ar (Ia)

H₂N-O-CH(R¹)-CH₂-O-Ar (Ib)

wobei R¹ für eine Alkylgruppe und Ar für die Phenylgruppe, welche nicht-aromatische Substituenten tragen kann, stehen, sowie den entsprechenden Salzgemischen.

Außerdem betrifft die Erfindung die Herstellung von Mischungen aus O-Phenoxyalkyloximen der allgemeinen Formeln VIa und VIb 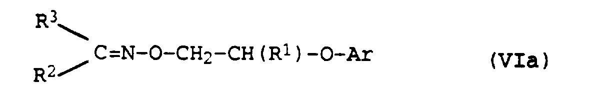 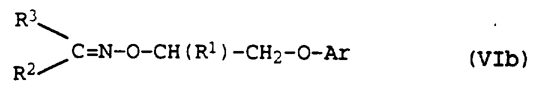 wobei R² eine Alkylgruppe und R³ eine Alkyl- oder Alkoxygruppe bedeuten, oder R² und R³ zusammen mit dem gemeinsamen C-Atom einen 5- bis 7-gliedrigen isocyclischen Ring bilden.

Ferner wurden bestimmte neue Isomerengemische gefunden.

Aus der älteren deutschen Anmeldung DE-A 42 44 390 ist bekannt, Oxime mit Phenoxyalkylierungsmitteln unter Mitverwendung einer Base zu O-Phenoxyalkyloximen umzusetzen.

Ferner ist aus der GB-A-2 115 812 ein Verfahren zur Herstellung von O-Phenoxyalkyloximen bekannt, bei dem das Mesylat oder Tosylat eines O-alkylierten Oxims mit einem Phenol mittels einer Base zur Reaktion gebracht wird.

Da die direkte Alkylierung des Hydroxylamins in der Regel nicht selektiv am O-Atom erfolgt, macht man sich bei den Verfahren zur Herstellung von O-Phenoxyalkylhydroxylaminen meist Schutzgruppen-Techniken zunutze (vgl. Houben-Weyl, Methoden der Organischen Chemie, Thieme Verlag, Stuttgart, 1990, Band E16a/1, Seite 214 bis Seite 250). Besondere Bedeutung hat dabei die Verwendung von N-Hydroxyphthalimid als N-geschütztes Hydroxylamin erlangt. Die gewünschten O-Phenoxyalkylhydroxylamine erhält man ausgehend von diesem durch Alkylierung der Hydroxygruppe und anschließende Abspaltung des Phthalsäureteils. Dieser Syntheseweg vermag jedoch aufgrund der niedrigen Ausbeuten und des Verlustes an der Schutzgruppenverbindung nicht zu befriedigen.

Der vorliegenden Erfindung lag daher ein wirtschaftlicheres Verfahren zur Herstellung von O-Phenoxyalkylhydroxylaminen und O-Phenoxyalkyloximen als Aufgabe zugrunde.

Demgemäß wurde ein Verfahren zur Herstellung von Mischungen aus O-Phenoxyalkylhydroxylaminen der Formel I gefunden, welches dadurch gekennzeichnet ist, daß man
a) ein Isomerengemisch aus O-(2-Hydroxyethyl)-oximen der Formeln IIa und IIb 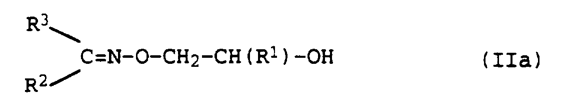 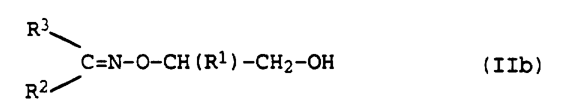 wobei R² eine Alkylgruppe und R³ eine Alkyl- oder Alkoxygruppe bedeuten, oder R² und R³ zusammen mit dem gemeinsamen C-Atom einen 5- bis 7-gliedrigen isocyclischen Ring bilden,
   mit einem Sulfonylhalogenid der Formel III

   Hal-SO₂-R⁴ (III)

   in der R⁴ für C₁-C₄ - Alkyl, C₁-C₄-Halogenalkyl oder C₁-C₄ - Alkylphenyl steht und Hal Halogen bedeutet, in Gegenwart einer Base in das entsprechende Sulfonatgemisch aus IVa und IVb 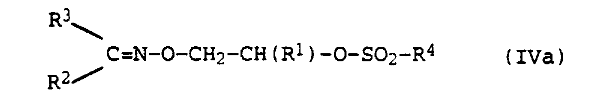 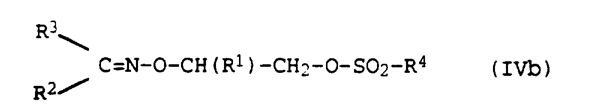 überführt,
b) dieses Sulfonatgemisch in Gegenwart einer Base mit einem Phenol der Formel V

   HO-Ar (V)

   zu einer Mischung aus O-Phenoxyalkyloximen der allgemeinen Formeln VIa und VIb 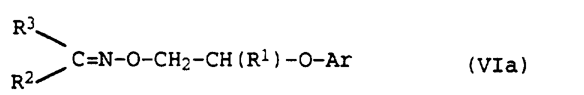 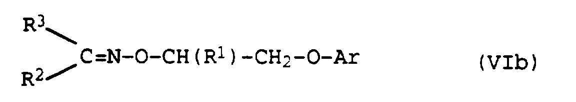 umsetzt,
c) diese Mischung in Gegenwart einer Säure hydrolysiert und gewünschtenfalls
d) aus den dabei anfallenden Salzen die O-Phenoxyalkylhydroxylamine Ia und Ib mittels Mineralbase freisetzt.

Ferner wurde ein Verfahren zur Herstellung von Mischungen aus O-Phenoxyalkyloximen der allgemeinen Formeln VIa und VIb gefunden, welches durch die vorstehend genannten Verfahrensschritte a) und b) gekennzeichnet ist.

Daneben erstreckt sich die Erfindung auf Isomerengemische aus
- {O-[2-(4-Chlorphenoxy)-propyl]-hydroxylammonium}-hydrogensulfat und {O-[2-(4-Chlorphenoxy)-1-methylethyl]-hydroxylammonium}-hydrogensulfat,
- O-[2-(4-chlorphenoxy)-propyl]-hydroxylammoniumchlorid und O-[2-(4-Chlorphenoxy)-1-methylethyl]-hydroxylammoniumchlorid,
- O-[2-Methylsulfonyloxypropyl]-propan-2-onoxim und O-[2-Methylsulfonyloxy-1-methylethyl]-propan-2-onoxim.

Das erfindungsgemäße Verfahren läßt sich in einem einfachen Fall wie folgt veranschaulichen:

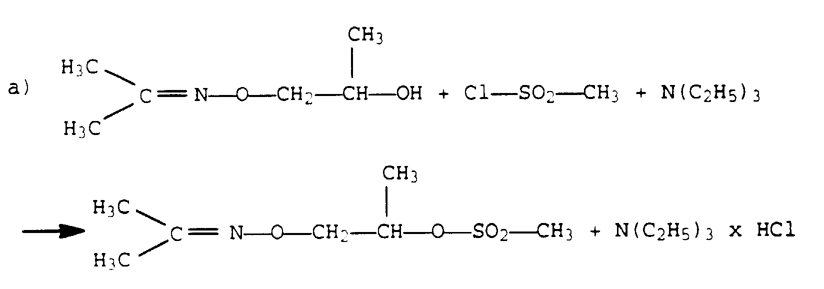

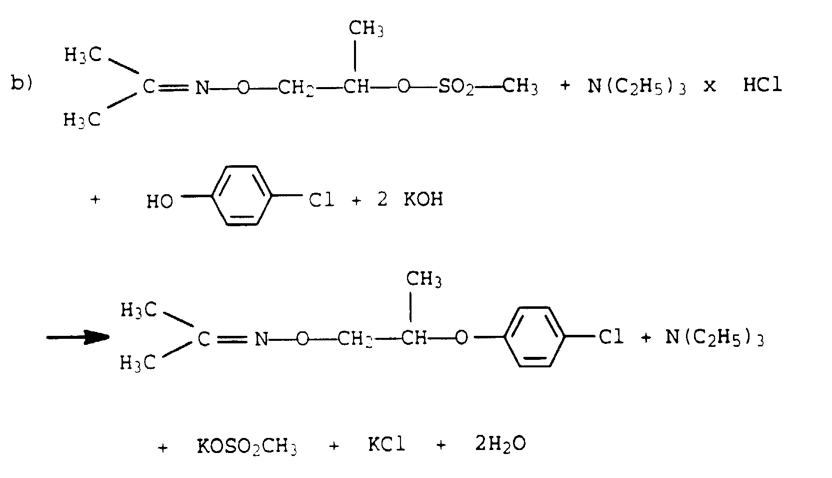

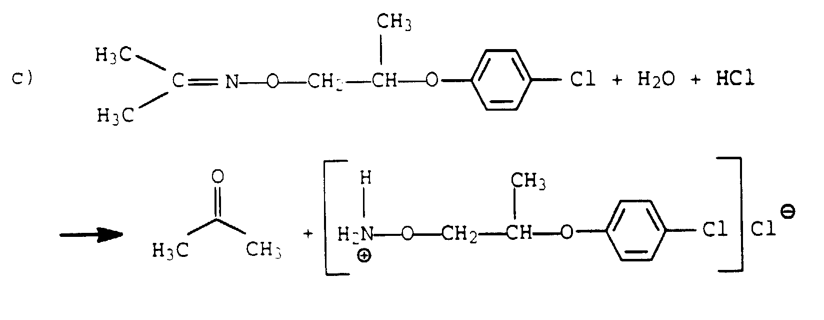

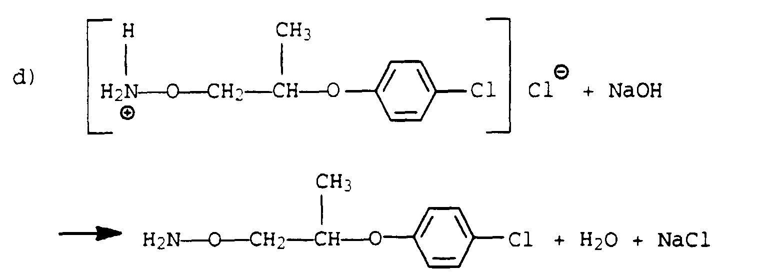

Im Hinblick auf die aus den Verfahrensprodukten VI und I herstellbaren Wirkstoffe haben die einzelnen Variablen vorzugsweise die folgende Bedeutung, und zwar sowohl für sich allein als auch in Kombination:
- Ar: die Phenylgruppe, welche unsubstituiert sein oder ein bis drei Substituenten tragen kann, jeweils ausgewählt aus der Gruppe bestehend aus Nitro, Cyano, Halogen, C₁-C₄-Alkyl oder C₁-C₄-Halogenalkyl, besonders bevorzugt 4-Halogenphenyl, insbesondere 4-Chlorphenyl;
- R²: eine C₁-C₄-Alkylgruppe, besonders bevorzugt Methyl; eine C₁-C₄-Alkylgruppe und
- R³: eine C₁-C₄-Alkyl- oder C₁-C₆-Alkoxygruppe
oder
- R² und R³: zusammen mit dem gemeinsamen C-Atom einen 5- bis 7-gliedrigen isocyclischen Ring.

Besonders bevorzugt sind diejenigen O-Phenoxyalkyloxime VI, in denen R¹ und R² für eine C₁-C₄-Alkylgruppe, vor allem die Methyl- und/oder die Ethylgruppe, daneben die n-Propyl- und die n-Butylgruppe stehen.

Ganz besonders bevorzugt sind diejenigen O-Phenoxyalkyloxime VI, welche sich von O-Phenoxyalkylacetonoxim ableiten, vor allem O-[2-(4-Chlorphenoxy)-propyl]-propan-2-onoxim.

Unter den O-Phenoxyalkylhydroxylaminen I sind diejenigen besonders bevorzugt, welche sich von den bevorzugten bzw. besonders bevorzugten O-Phenoxyalkyloximen VI, wie vorstehend genannt, ableiten.

Die Sulfonate IV sind durch Umsetzung von 0-(2-Hydroxyalkyl)-oximen II mit Sulfonylhalogeniden III in Gegenwart von Basen erhältlich.

Die O-(2-Hydroxyalkyl)-oxime II sind bekannt oder können nach bekannten Methoden, beispielsweise durch Umsetzung der entsprechenden Ketoxime VII mit Alkylenoxiden VIII {vgl. z.B. U.S. 3,040,097; J. Am. Chem. Soc. 81, Seiten 4223 ff. (1959)} oder den entsprechenden Carbonaten IX, hergestellt werden. Dabei werden in der Regel Isomerengemische aus IIa und IIb erhalten. 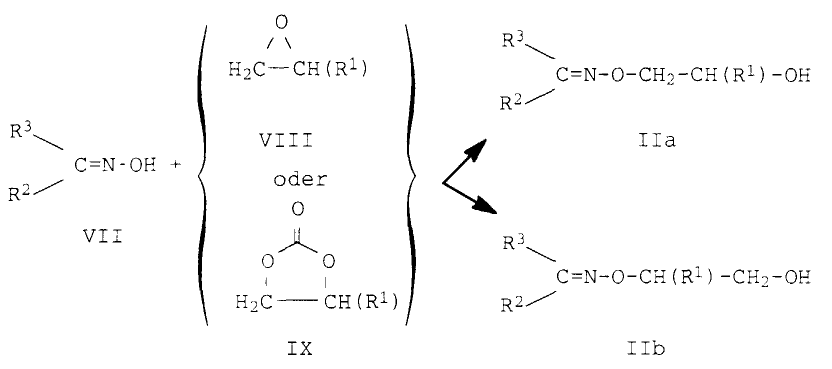 Als Sulfonylhalogenide III eignen sich vorzugsweise solche, in denen R⁴ eine C₁-C₄-Alkyl-, C₁-C₄-Halogenalkyl- oder C₁-C₄-Alkylphenylgruppe, und Hal vorzugsweise Chlor und daneben Brom bedeutet, vor allem Methylsulfonylchlorid, Trifluormethylsulfonylchlorid und 4-Methylphenylsulfonylchlorid.

Die Sulfonylhalogenide III sind kommerziell erhältlich oder können nach bekannten Methoden, z.B. durch Umsetzung der entsprechenden organischen Sulfonsäuren mit anorganischen Halogenierungsmitteln wie Phosphorpentachlorid hergestellt werden.

Das Molverhältnis von O-(2-Hydroxyalkyl)-oxim II (also die Summe aus IIa + IIb) zu Sulfonylhalogenid III beträgt vorzugsweise 1:1 bis 1:1,5, insbesondere 1:1 bis 1:1,2.

Die Umsetzung kann lösungsmittelfrei oder vor allem in einem organischen Lösungsmittel durchgeführt werden.

Geeignete organische Lösungsmittel sind beispielsweise aliphatische Kohlenwasserstoffe wie n-Alkane mit mehr als 4-C-Atomen, vor allem n-Pentan und n-Hexan, Cycloalkane wie Cyclohexan, aromatische Kohlenwasserstoffe, vor allem Toluol und die Xylole, dipolar aprotische Lösungsmittel wie Ether, vor allem Diethylether, Tetrahydrofuran und 1,4-Dioxan, Sulfoxide wie Dimethylsulfoxid und Diethylsulfoxid, Sulfone wie Dimethylsulfon, Diethylsulfon und Tetramethylensulfon, Nitrile wie Acetonitril und Benzonitril, N,N-disubstituierte Carbonsäureamide wie Dimethylformamid, N,N-Dimethylbenzamid und N,N-Dimethylacetamid, N-Alkyllactame wie N-Methylpyrrolidon und N-Butylpyrrolidon, tetrasubstituierte cyclische und acyclische Harnstoffe wie N,N,N',N'-Tetramethyl- und -n-butylharnstoff, sowie Gemische der genannten Solventien.

Die Umsetzung kann aber auch in einem Überschuß einer geeigneten Aminbase als Lösungsmittel durchgeführt werden.

Das Lösungsmittel bzw. Lösungsmittelgemisch wird im allgemeinen in einer Menge von 0,1 bis 2, vorzugsweise 0,3 bis 1 kg pro Mol des O-(2-Hydroxyalkyl)-oxims II eingesetzt.

Als Basen eignen sich anorganische und vorzugsweise organische Basen, vor allem aliphatische tertiäre Amine wie Triethylamin, Tri-n-butylamin und N,N-Dimethylcyclohexylamin und daneben aromatische tertiäre Amine wie Pyridin und 1,3-Pyrimidin.

Pro Mol des O-(2-Hydroxyalkyl)-oxims II verwendet man zweckmäßigerweise 1 bis 2, insbesondere 1 bis 1,5 Äquivalente der Base. Verwendet man die Base gleichzeitig als Lösungsmittel, so liegt diese normalerweise in einem noch größeren Überschuß vor.

Verfahrenstechnisch geht man zweckmäßigerweise so vor, daß man das Isomerengemisch aus IIa und IIb, die Base und gegebenenfalls das Lösungsmittel vorlegt, danach das Sulfonylhalogenid III zusetzt und das Gemisch auf die Reaktionstemperatur erhitzt.

Die Reaktion gelingt in der Regel bei Temperaturen von (-20) bis 100, vorzugsweise 0 bis 40 °C und Drücken von 0,5 bis 2 bar. Vorzugsweise arbeitet man bei Normaldruck. Üblicherweise haben die Ausgangsprodukte nach 0,5 bis 3 Stunden vollständig abreagiert.

Zur Aufarbeitung versetzt man das Reaktionsgemisch zweckmäßigerweise mit Wasser und isoliert, meist unter Zuhilfenahme eines organischen Lösungsmittels wie Toluol oder Diethylether, eine organische Phase, welche aus den Sulfonaten IVa und IVb besteht oder diese enthält.

Das Lösungsmittel entfernt man vorzugsweise destillativ, und es können sich hieran weitere an sich bekannte Reinigungsoperationen wie Destillation oder Umkristallisation anschließen, wodurch man die Sulfonate IV (als gereinigte Mischung aus IVa und IVb oder die getrennten Isomere) in reiner Form erhält.

In einer bevorzugten Ausführungsform des erfindungsgemäßen Verfahrens wird die organische Phase nach der Extraktion als solche in die Folgestufe übergeführt, wo das Sulfonatgemisch aus IVa und IVb mit einem Phenol V unter Mitverwendung einer Base zu einer Mischung aus O-Phenoxyalkyloximen VIa und VIb umgesetzt wird.

In einer besonders bevorzugten Ausführungsform des erfindungsgemäßen Verfahrens setzt man das rohe Reaktionsgemisch als solches in der Folgestufe ein.

Die Phenole V sind kommerziell erhältlich oder können nach bekannten Methoden bzw. analog zu diesen hergestellt werden.

Pro Mol des Sulfonats IV (also der Summe aus IVa und IVb) verwendet man zweckmäßigerweise 1 bis 1,5 mol und insbesondere äquimolare Mengen des Phenols V.

Als Base verwendet man vorzugsweise Mineralbasen, vor allem Erdalkalimetallhydroxide oder Alkalimetallhydroxide, insbesondere Kaliumhydroxid, und daneben Natriumhydroxid oder Calciumhydroxid. Ferner eignen sich Natriumcarbonat, Kaliumcarbonat, Natriumhydrogencarbonat und Kaliumhydrogencarbonat.

Pro Mol Sulfonat IV (IVa + IVb) werden in der Regel 1 bis 5, vorzugsweise 1,5 bis 3 mol der Base verwendet.

Die Umsetzung kann lösungsmittelfrei oder in einem organischen Lösungsmittel/Lösungsmittelgemisch durchgeführt werden.

Als organisches Lösungsmittel eignen sich vor allem Methanol, Ethanol und Isopropanol oder eines der bei der Verfahrensstufe (a) genannten Lösungsmittel.

Man setzt das Lösungsmittel oder Lösungsmittelgemisch im allgemeinen in einer Menge von bis zu 2, vorzugsweise bis zu 1 kg pro Mol des Sulfonats IV ein.

In der Regel gelingt die Reaktion bei Temperaturen von 20 bis 180°C, vorzugsweise 40 bis 100 °C. Die Reaktionsdauer liegt meist bei etwa 1 bis 20 Stunden.

Bei der Aufarbeitung des Reaktionsgemisches auf das O-Phenoxyalkyloxim I destilliert man in der Regel zunächst die flüchtigen Bestandteile, vor allem Wasser und gegebenenfalls das Lösungsmittel, ab und reinigt das rohe O-Phenoxyalkyloxim I gewünschtenfalls mittels an sich bekannter Reinigungsoperationen wie Umkristallisieren, Digerieren oder Extrahieren unter Verwendung eines organischen Lösungsmittels weiter.

Die Ausbeuten an den O-Phenoxyalkyloximen VI (VIa und VIb zusammen) liegen meist zwischen 60 und 90%.

Wird die Mischung aus O-Phenoxyalkyloximen VIa und VIb unmittelbar nach ihrer Herstellung der sauren Hydrolyse zu den Salzen von Ia und Ib unterworfen, so kann in aller Regel eine Reinigung unterbleiben.

Als Säuren für die Hydrolyse eignen sich vorzugsweise Protonensäuren, vor allem Schwefelsäure und Chlorwasserstoffsäure, daneben Phosphorsäure, Trifluoressigsäure und Methansulfonsäure, und zwar insbesondere in Form ihrer wäßrigen Lösungen.

Die Säuren werden in der Regel im Molverhältnis von 1:1 bis 10:1, zweckmäßigerweise von 5:1 bis 7:1, bezogen auf das O-Phenoxyalkyloxim VI (also der Summme aus VIa und VIb), verwendet.

Die saure Spaltung der Mischung aus VIa und VIb nimmt man zweckmäßigerweise in einem inerten Lösungsmittel, vor allem in Wasser vor.

Die Reaktion erfolgt meist bei Temperaturen von 50 bis 130, vorzugsweise 60 bis 80°C und Drücken von 0,1 bis 1, vorzugsweise von 0,3 bis 0,5 bar. Sie kann diskontinuierlich oder kontinuierlich durchgeführt werden. Die Reaktionszeiten betragen in der Regel etwa 4 bis 24, vor allem 6 bis 12 Stunden.

Aus dem rohen Reaktionsgemisch wird bei Reaktionsende in der Regel zunächst das freigesetzte Keton (R²-CO-R³) und gegebenenfalls das Lösungsmittel, vor allem destillativ, entfernt.

In einer bevorzugten Ausführungsform des erfindungsgemäßen Verfahrens, die auch für sich allein besonders vorteilhaft ist, geht man von einer Mischung aus VIa und VIb aus, in welchen die Reste R² und R³ Methyl bedeuten und hydrolysiert diese in Gegenwart von Schwefelsäure oder Salzsäure. Das dabei entstehende Aceton entfernt man zweckmäßigerweise laufend aus dem Reaktionsgemisch, wobei auch ein Teil des Wassers, und bei Verwendung von Salzsäure auch Chlorwasserstoff mit übergehen kann.

Danach wird das rohe Reaktionsgemisch in an sich bekannter Weise auf die Salze der O-Phenoxyalkylhydroxylamine Ia und Ib aufgearbeitet.

Bei Verwendung von Salzsäure erfolgt die Aufarbeitung zweckmäßigerweise so, daß man Chlorwasserstoff und Wasser, die bei Reaktionsende noch im Reaktionsgefäß vorliegen, weitestgehend durch azeotrope Destillation zusammen entfernt.

Die zurückgewonnene überschüssige Säure läßt sich, insbesondere im Falle der Schwefelsäure und der Salzsäure, in die Spaltung der O-Phenoxyalkyloxime VIa/VIb zurückführen.

Danach nimmt man die verbleibenden sauren Salze der O-Phenoxyalkylhydroxylamine Ia und Ib normalerweise in Wasser auf.

Aus der wäßrigen Salzlösung können die Salze von Ia und Ib gewünschtenfalls in an sich bekannter Weise als solche erhalten werden, z.B. durch Einengen der Lösung und Auskristallisieren.

Bei Verwendung von 30 bis 60 gew.-%iger Schwefelsäure bei der Hydrolyse tritt die Kristallisation der Hydrogensulfate von Ia und Ib in der Regel schon beim Abkühlen des Reaktionsgemisches auf eine Temperatur um 20 °C ein.

Das derart gefällte Salz des O-Phenoxyalkylhydroxylamins Ia/Ib trennt man von der überstehenden Lösung wie üblich ab, insbesondere durch Abfiltrieren und Waschen der Kristalle, z.B. mit Wasser und Toluol.

Ia und Ib, zusammen oder nach deren Trennung, werden aus ihren Salzen mittels einer Mineralbase freigesetzt.

Als Mineralbasen eignen sich insbesondere Alkalimetall- und Erdalkalimetallhydroxide, -carbonate und -hydrogencarbonate, wobei die Hydroxide bevorzugt, und Natrium-, Kalium- oder Calciumhydroxid besonders bevorzugt sind.

Pro Mol der O-Phenoxyalkylhydroxylamine I (also der Summme aus Ia und Ib) werden zweckmäßigerweise 1 bis 3, insbesondere 1,1 bis 1,5 Äquivalente an Mineralbase verwendet.

Die Neutralisation wird in an sich bekannter Weise durchgeführt, so daß sich nähere Erläuterungen hierzu erübrigen. Dabei erhält man normalerweise eine organische Phase, welche die O-Phenoxyalkylhydroxylamine Ia und Ib enthält oder aus diesen besteht, sowie eine salzhaltige wäßrige Phase.

Die Aufarbeitung auf die einzelnen O-Phenoxyalkylhydroxylamine Ia oder Ib erfolgt nach den hierfür bekannten Techniken, vor allem durch Phasentrennung, Extraktion der wäßrigen Phase mit einem organischen Lösungsmittel wie Toluol oder Diethylether, Vereinigen der organischen Phasen und anschließendes, vor allem destillatives, Entfernen des Lösungsmittels. Hieran können sich gewünschtenfalls weitere an sich bekannte Reinigungsoperationen wie Destillation oder Umkristallisation anschließen.

Die Ausbeuten an O-Phenoxyalkylhydroxylaminen I (Ia und Ib zusammen) liegen meist bei etwa 80 bis 90 %.

Das erfindungsgemäße Verfahren bietet den besonderen Vorteil, daß über den Verlauf der Stufen a), b) und c) eine Abreicherung des b-Isomeren erfolgt, das im Hinblick auf die aus I herstellbaren Wirkstoffe oft unerwünscht ist. So wurde gefunden, daß ausgehend von Gemischen aus IIa und IIb, in denen der Anteil des Isomeren IIa zwischen 70 und 100% liegt und insbesondere 70 bis 99,5, und vor allem 70 bis 98% beträgt, die erhalten Isomerengemische aus Ia und Ib einen um 75 bis 98% geringeren Anteil des b-Isomeren enthalten.

Weiterhin wurde gefunden, daß diese Abreicherung des b-Isomeren in der Regel zum Teil bereits in den Stufen a) und b) - also bei der Herstellung der Mischungen aus VIa und VIb - erfolgt und sich häufig noch verbessern läßt, wenn man deren saure Hydrolyse mittels Schwefelsäure vornimmt und danach, oder im Anschluß an die Hydrolyse bei Verwendung einer anderen Mineralsäure, aus den so erhaltenen Lösungen die O-Phenoxyalkylhydroxylamine Ia und Ib als Sulfate ausfällt.

Mit dem vorliegenden Verfahren erübrigt sich die Trennung der Isomeren IIa und IIb. Da die aus dem Verfahren resultierenden Mischungen bezüglich VIa oder Ia, den bevorzugten Zielprodukten, angereichert sind, ist deren Reinigung weit weniger aufwendig, sofern sie nicht ganz entfallen kann.

Die Verfahrensprodukte I bzw. VI eignen sich als Vorprodukte für Herbizide vom Cyclohexenon-Typ (vgl. z.B. EP-A 456 112).

### Ausführungsbeispiele

### Beispiel 1

Herstellung von O-[2-(4-chlorphenoxy)-propyl]-propan-2-onoxim und O-[2-(4-Chlorphenoxy)-propyl]-hydroxylamin (Ia, VIa; R¹, R², R³ = Methyl; Ar = 4-Chlorphenyl)

### Variante 1:

a) Herstellung der O-Sulfonyloxyalkyloxime (IV)
   57,3 kg (437 mol) eines Gemisches aus 91,2 Gew.-% O-(2-Hydroxy-propyl)-propan-2-onoxim und 8,7 Gew.-% O-(2-Hydroxy-1-methylethyl)-propan-2-onoxim wurden in 66,3 kg (656 mol) Triethylamin und 175 kg Toluol vorgelegt. Zu der Mischung wurden bei 10 bis 20 °C 55,1 kg (481 mol) Methylsulfonylchlorid gegeben, und das Ganze wurde noch 1 Stunde bei 25 °C nachgerührt. Eine Probe des Rohprodukts wurde auf die beiden Hauptprodukte aufgearbeitet:
   O-[2-Methylsulfonyloxypropyl]-propan-2-onoxim (n_{D}²² = 1,4538):
   ¹H-NMR (400,1 MHz; in d⁶-Dimethylsulfoxid): δ [ppm] = 4,88 (1H,m); 4,05 (2H,d); 3,1 (3H,s); 1,83 (3H,s); 1,82 (3H,s); 1,3 (3H,d).
   ¹³C-NMR (100,6 MHz; in d⁶-Dimethylsulfoxid): δ [ppm] = 155,4 (tert. C); 77,6 (CH); 74,7 (CH₂); 37,8 (CH₃); 21,2 (CH₂); 17,4 (CH₃); 15,4 (CH₃).
   O-[2-Methylsulfonyloxy-1-methylethyl]-propan-2-onoxim (n_{D}²² = 1,4520):
   ¹H-NMR (270,1 MHz; in d⁶-Dimethylsulfoxid): δ [ppm] = 4,3 (1H,m); 4,25 (2H,m); 3,15 (3H,s); 1,82 (3H,s); 1,77 (3H,s); 1,2 (3H,d).
   ¹³C-NMR (125,7 MHz; in d⁶-Dimethylsulfoxid): δ [ppm] = 154,9 (tert. C); 75,0 (CH); 71,3 (CH₂); 36,6 (CH₃); 21,3 (CH₃); 15,7 (CH₃); 15,3 (CH₃).
b) Herstellung der O-Phenoxyalkyloxime (VI)
   Anschließend wurde unter Rühren eine Lösung von 56,4 kg (439 mol) 4-Chlorphenol in 147 kg 40%-iger (1050 mol) Kalilauge zugesetzt. Toluol und Triethylamin wurden danach bei 0,2 bar und 60 °C abdestilliert und die Reaktionsmischung wurde noch 16 Stunden bei dieser Temperatur gerührt.
   Nach dem Vermischen des Rohprodukts mit 150 1 Wasser wurden wäßrige und organische Phase getrennt, und die Wasserphase wurde mit insgesamt 150 1 Toluol extrahiert. Die vereinigten organischen Phasen wurden noch einmal mit 150 1 Wasser gewaschen. Nach destillativem Entfernen des Lösungsmittels aus der organischen Phase bei vermindertem Druck blieben 71,9 kg eines Öls zurück, welches zu ca. 96,3 Gew.-% aus einem Gemisch von 67,2 kg (278 mol) O-[2-(4-Chlorphenoxy)-propyl]-propan-2-onoxim (Ausbeute: 70 %) und 2,1 kg (9 mol) O-[2-(4-chlorphenoxy)-1-methylethyl]-propan-2-onoxim bestand und welches bei 38 °C erstarrte (Isomerenverhältnis VIa/VIb = 97:3).
c) Saure Spaltung der O-Phenoxyalkyloxime
   71,9 kg dieses Gemisches wurden mit 350 kg 40%iger (1430 mol) Schwefelsäure versetzt. Danach wurde die Reaktionsmischung auf 80 °C erhitzt, und bei 300 mbar wurden über einen Zeitraum von 12 Stunden 177 kg Aceton und Wasser abdestilliert, wobei das dem Gemisch entzogene Wasser laufend ersetzt wurde, um die Säurekonzentration im Reaktionsgefäß konstant zu halten. Beim Abkühlen der verbliebenen Lösung auf 20 °C fielen 60,9 kg Niederschlag aus, welcher zu 99,5 Gew.-% aus dem Bisammonium-Sulfat von O-[2-(4-Chlorphenoxy)-propyl]-hydroxylamin (Anteil: 98,9 Gew.-%; Ausbeute: 85%) und von O-[2-(4-Chlorphenoxy)-1-methylethyl]-hydroxylamin (0,6 Gew.-%) bestand. Dieser Niederschlag wurde nacheinander mit jeweils 175 kg Wasser und Toluol gewaschen, und die so erhaltenen Kristalle wurden bei 40 °C im Vakuum getrocknet (Isomerenverhältnis = 99,4:0,6).
   Bis-{O-[2-(4-Chlorphenoxy)-propyl]-hydroxylammonium}-sulfat:
   ¹H-NMR (250,1 MHz; in d⁶-Dimethylsulfoxid): δ [ppm] = 8,55 (3H,s); 7,33 (2H,d); 7,0 (2H,d); 4,7 (1H,m); 3,9 (2H,m); 1,22 (3H,d).
   ¹³C-NMR (125,7 MHz; in d⁶-Dimethylsulfoxid): δ [ppm] = 156,4 (tert. C); 129,4 (2CH); 124,5 (tert. C); 117,6 (2CH); 77,2 (CH₂); 71,6 (CH); 16,2 (CH₃).
d) Freisetzung der O-Phenoxyalkylhydroxylamine (I)
   Zu dem Rohprodukt wurden unter Rühren 50 kg 25%iger (312,5 mol) Natronlauge gegeben. Die organische Phase wurde abgetrennt, die wäßrige Phase mit 50 kg Toluol extrahiert und von den vereinigten organischen Phasen das Toluol abdestilliert. Zurück blieben 48,3 kg eines Isomerengemisches von O-[2-(4-Chlorphenoxy)-propyl]-hydroxylamin (Ia; 99,4 Gew.-%,; Ausbeute: 99%) und O-[2-(4-Chlorphenoxy)-1-methylethyl]-hydroxylamin (Ib; 0,6 Gew.-%).
   Die Ausbeute an O-[2-(4-Chlorphenoxy)-propyl]-hydroxylamin betrug 59 %, bezogen auf eingesetztes O-(2-Hydroxy-propyl)-propan-2-onoxim.

### Beispiel 2

Es wurde wie in Beispiel 1 von einem Gemisch aus O-(2-Hydroxypropyl)-propan-2-onoxim und O-(2-Hydroxy-1-methylethyl)-propan-2-onoxim, jedoch im Verhältnis 99,4 : 0,6, ausgegangen und ansonsten analog zu Beispiel 1 gearbeitet. Dabei wurden O-[2-(4-Chlorphenoxy)-propyl]-hydroxylamin (Ausbeute: 54%) und O-[2-(4-Chlorphenoxy)-1-methylethyl]-hydroxylamin im Verhältnis 99,98 : 0,02 erhalten.

### Beispiel 3

Es wurde wie in Beispiel 1 von einem Gemisch aus O-(2-Hydroxypropyl)-propan-2-onoxim und O-(2-Hydroxy-1-methylethyl)-propan-2-onoxim, jedoch im Verhältnis 76,2 : 23,8, ausgegangen und ansonsten analog zu Beispiel 1 gearbeitet. Dabei wurden O-[2-(4-Chlorphenoxy)-propyl]-hydroxylamin (Ausbeute: 56%) und O-[2-(4-Chlorphenoxy)-1-methylethyl]-hydroxylamin im Verhältnis 98,5 : 0,6 erhalten.

### Beispiel 4

### Herstellung von O-(2-Phenoxybutyl)-hydroxylamin (VIa; R¹ = Ethyl; R², R³ = Methyl; Ar = 4-Hydroxyphenyl)

Es wurde von einem Gemisch aus O-(2-Hydroxybutyl)-propan-2-onoxim und O-(2-Hydroxy-1-ethylethyl)-propan-2-onoxim im Verhältnis 96,7 : 3,3 ausgegangen. Anstelle von 4-Chlorphenol wurde Phenol verwendet und im übrigen wurde analog zu Beispiel 1 gearbeitet. Dabei wurden O-(2-Phenoxybutyl)-hydroxylamin (Ausbeute: 50 %) und O-(2-Phenoxy-1-ethylethyl)-hydroxylamin im Verhältnis 99,8 : 0,2 erhalten.

### Beispiel 5

### Herstellung von O-[2-(4-Chlorphenoxy)-propyl]-hydroxylamin (VIa; R¹, R² = Methyl; R³ = Ethyl; Ar = 4-Chlorphenyl)

Es wurde von einem Gemisch aus O-(2-Hydroxypropyl)-butan-2-onoxim und O-(2-Hydroxy-1-methylethyl)-butan-2-onoxim im Verhältnis 92,7 : 7,3 ausgegangen. Im übrigen wurde analog zu Beispiel 1 gearbeitet. Dabei wurden O-[2-(4-Chlorphenoxy)-propyl]-hydroxylamin (Ausbeute: 25%) und O-[2-(4-Chlorphenoxy)-1-methylethyl]-hydroxylamin im Verhältnis 99,9 : 0,1 erhalten.

### Beispiel 6

Herstellung von O-[2-(4-chlorphenoxy)-propyl]-propan-2-onoxim und O-[2-(4-Chlorphenoxy)-propyl]-hydroxylamin (Ia, VIa; R¹, R², R³ = Methyl; Ar = 4-Chlorphenyl)

### Variante 2:

a) Herstellung der O-Sulfonyloxyalkyloxime
   4 kg (29 mol) eines Gemisches aus 72,6 Gew.-% O-(2-Hydroxypropyl)-propan-2-onoxim und 22,7 Gew.-% O-(2-Hydroxy-1-methylethyl)-propan-2-onoxim (Isomerenverhältnis IIa/IIb = 76,2:23,8) wurden in 4,6 kg (36 mol) N,N-Dimethylcyclohexylamin und 20 kg o-Xylol vorgelegt. Diese Mischung wurde bei 5 bis 20 °C mit einer Lösung von 3,8 kg (33 mol) Methylsulfonylchlorid in 5 kg o-Xylol versetzt, und das Ganze wurde noch 1 Stunde bei 25 °C nachgerührt.
b) Herstellung der O-Phenoxyalkyloxime
   Nach Vermischen des Rohprodukts mit 7,5 kg Wasser wurde die organische Phase abgetrennt und diese dann mit 15 kg Isopropanol, 3,9 kg (30 mol) 4-Chlorphenol und 8,4 kg 40%iger (59 mol) Kalilauge 10 Stunden auf 80 °C erhitzt. Danach wurde der überwiegende Teil des iso-Propanols und des Wassers bei vermindertem Druck abdestilliert, und es bildeten sich im Sumpf zwei Phasen. Aus der abgetrennten organischen Phase wurde das o-Xylol weitestgehend abdestilliert und man erhielt 4,92 kg eines Öls, welches zu 93,8 Gew.-% aus einem Gemisch von 93,6 Gew.-% (17,9 mol) O-[2-(4-Chlorphenoxy)-propyl]-propan-2-onoxim (Ausbeute: 85 %) und 6,4 Gew.-% (1,2 mol) O-[2-(4-Chlorphenoxy)-1-methylethyl]-propan-2-onoxim bestand.
c1) Saure Spaltung der O-Phenoxyalkyloxime
   160 g eines Gemisches, bestehend zu 96,9 Gew.-% (0,64 mol) aus O-[2-(4-chlorphenoxy)-propyl]-propan-2-onoxim und zu 3,1 Gew.-% (0,02 mol) aus O-[2-(4-Chlorphenoxy)-1-methylethyl]-propan-2-on-oxim (Isomerenverhältnis VIa/VIb = 97:3) wurden mit 730 g 21%-iger (4,2 mol) Salzsäure versetzt. Bei 80 °C und 315 mbar wurden unter einem Rücklaufverhältnis von 12:1 202 g einer vorwiegend aus Wasser und Aceton bestehenden Flüssigkeit abdestilliert. Anschließend wurden ohne Rücklauf bei 68 °C und 180 mbar 440 g azeotrop destillierende Salzsäure abdestilliert. Der Rückstand (184 g) enthielt 70,6 Gew.-% des Hydrochlorids von O-[2-(4-Chlorphenoxy)-propyl]-hydroxylamin (Ausbeute: 85 %) neben 1,1 Gew.-% des Hydrochlorids von O-[2-(4-Chlorphenoxy)-1-methylethyl]-hydroxylamin (Isomerenverhältnis Ia/Ib = 98,5:1,5).
   O-[2-(4-Chlorphenoxy)-propyl]-hydroxylammoniumchlorid:
   ¹H-NMR (270,1 MHz; in d⁶-Dimethylsulfoxid): δ [ppm] = 11,25 (3H,s); 7,3 (2H,d); 7,05 (2H,d); 4,85 (1H,m); 4,25 (2H,m); 1,25 (3H,d).
   ¹³C-NMR (125,7 MHz; in d6-Dimethylsulfoxid): δ [ppm] = 156,1 (tert. C); 129,4 (2 CH); 124,8 (tert. C); 117,7 (2 CH); 76,4 (CH₂); 71,3 (CH); 15,9 (CH₃).
c2) Umfällung mit Schwefelsäure
   Zu diesem Rückstand wurden 120 g Wasser, 124 g 25%ige (775 mmol) Natronlauge und 126 g Toluol gegeben; nach dem Vermischen wurde die organische Phase abgetrennt und mit 76 g (2,38 mol) Methanol und 64 g 50%iger (327 mmol) Schwefelsäure versetzt, woraufhin 132,8 g Bisammonium-Sulfate von O-[2-(4-Chlorphenoxy)-propyl]-hydroxylamin (98,9 Gew.-%) und O-[2-(4-Chlorphenoxy)-1-methylethyl]-hydroxylamin (1,1 Gew.-%) ausfielen. Sie wurden abfiltriert, mit 190 g Toluol gewaschen und danach getrocknet (82 % Ausbeute bezogen auf das verwendete [2-(4-Chlorphenoxy)-propyl]-propan-2-onoxim; Isomerenverhältnis 98,9:1,1).
d) Freisetzung der O-Phenoxyalkylhydroxylamine
   Die Freisetzung der Hydroxylamine erfolgte analog zu Beispiel 1. Die Ausbeute an O-[2-(4-Chlorphenoxy)-propyl]-hydroxylamin betrug 98 %; das Isomerenverhältnis bezogen auf O-[2-(4-Chlorphenoxy)-1-methylethyl]-hydroxylamin war 98,1:1,1.

## Patentansprüche

1. Verfahren zur Herstellung von Isomerengemischen aus O-Phenoxyalkylhydroxylaminen der allgemeinen Formeln Ia und Ib
H₂N-O-CH₂-CH(R¹)-O-Ar (Ia)
H₂N-O-CH(R¹)-CH₂-O-Ar (Ib)
wobei R¹ für eine Alkylgruppe und Ar für die Phenylgruppe, welche nicht-aromatische Substituenten tragen kann, stehen, sowie den entsprechenden Salzgemischen, dadurch gekennzeichnet, daß man
a) ein Isomerengemisch aus O-(2-Hydroxyethyl)-oximen der Formeln IIa und IIb 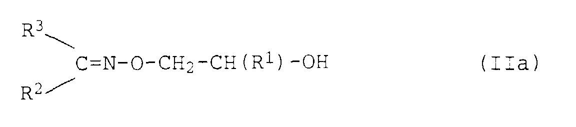 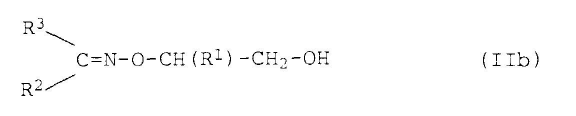 wobei R² eine Alkylgruppe und R³ eine Alkyl- oder Alkoxygruppe bedeuten, oder R² und R³ zusammen mit dem gemeinsamen C-Atom einen 5- bis 7-gliedrigen isocyclischen Ring bilden,
mit einem Sulfonylhalogenid der Formel III
Hal-SO₂-R⁴ (III)
in der R⁴ für C₁-C₄-Alkyl, C₁-C₄-Halogenalkyl oder C₁-C₄-Alkylphenyl steht und Hal Halogen bedeutet, in Gegenwart einer Base in das entsprechende Sulfonatgemisch aus IVa und IVb 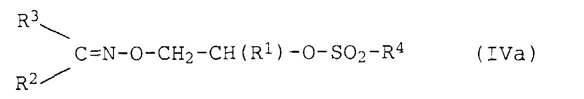 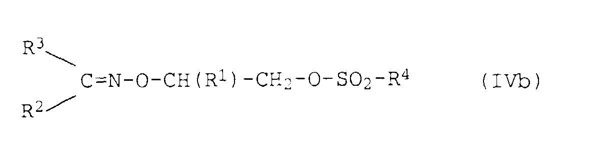 überführt,
b) dieses Sulfonatgemisch in Gegenwart einer Base mit einem Phenol der Formel V
HO-Ar (V)
zu einer Mischung aus O-Phenoxyalkyloximen der allgemeinen Formeln VIa und VIb 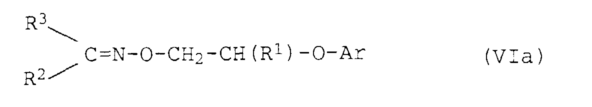 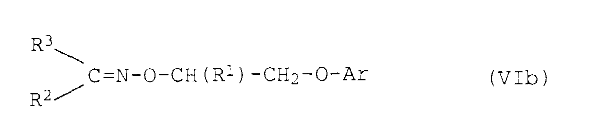 umsetzt,
c) diese Mischung in Gegenwart einer Säure hydrolysiert und gewünschtenfalls
d) aus den dabei anfallenden Salzen die O-Phenoxyalkylhydroxylamine Ia und Ib mittels Mineralbase freisetzt.

2. Verfahren zur Herstellung von Mischungen aus O-Phenoxyalkyloximen der allgemeinen Formeln VIa und VIb 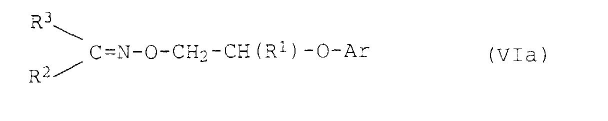 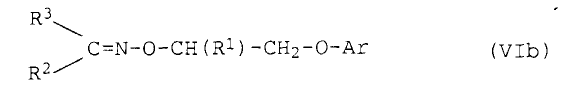 wobei R¹ bis R³ und Ar die in Anspruch 1 gegebenen Bedeutungen haben, dadurch gekennzeichnet, daß man
a) ein Isomerengemisch aus O-(2-Hydroxyethyl)-oximen der Formeln IIa und IIb 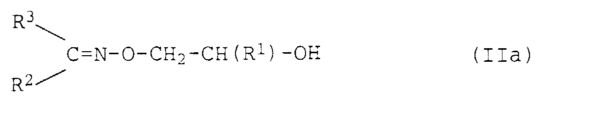 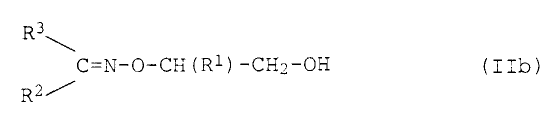 mit einem Sulfonylhalogenid der allgemeinen Formel III
Hal-SO₂-R⁴ (III)
in der R⁴ für C₁-C₄-Alkyl, C₁-C₄-Halogenalkyl oder C₁-C₄-Alkylphenyl steht und Hal Halogen bedeutet, in Gegenwart einer Base in das entsprechende Sulfonatgemisch aus IVa und IVb 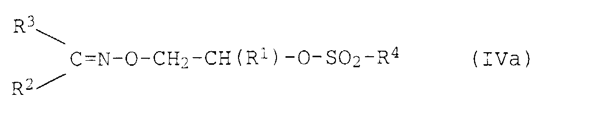 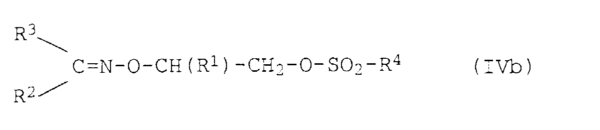 überführt und
b) dieses Sulfonatgemisch in Gegenwart einer Base mit einem Phenol der Formel V
HO-Ar (V)
umsetzt.

3. Verfahren zur Herstellung von Isomerengemischen aus O-Phenoxyalkylhydroxylaminen der Formeln Ia und Ib
H₂N-O-CH₂-CH(R¹)-O-Ar (Ia)
H₂N-O-CH(R¹)-CH₂-O-Ar (Ib)
wobei R¹ und Ar die in Anspruch 1 gegebenen Bedeutungen haben, sowie den entsprechenden Salzgemischen,
dadurch gekennzeichnet, daß man eine Mischung der O-Phenoxyalkyloxime VIa und VIb 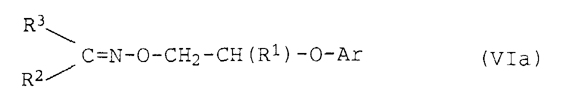 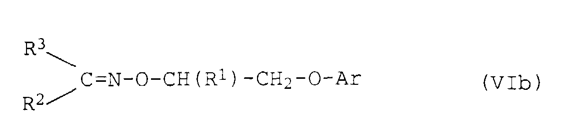 wobei R² eine Alkylgruppe und R³ eine Alkyl- oder Alkoxygruppe bedeuten, oder R² und R³ zusammen mit dem gemeinsamen C-Atom einen 5- bis 7-gliedrigen isocyclischen Ring bilden,
in Gegenwart einer Säure hydrolysiert und die hierbei anfallenden Salze von Ia und Ib gewünschtenfalls mit einer Base umsetzt.

4. Verfahren nach Anspruch 1 oder 2, dadurch gekennzeichnet, daß man als Base in der Verfahrensstufe (a) ein aliphatisches tertiäres Amin, und in der Verfahrensstufe (b) eine Mineralbase einsetzt.

5. Verfahren nach Anspruch 1 oder 2, dadurch gekennzeichnet, daß man das Sulfonatgemisch aus IVa und IVb, ohne es zu isolieren, mit dem Phenol V zu einer Mischung aus O-Phenoxyalkyloximen Ia und Ib umsetzt.

6. Verfahren nach Anspruch 1 oder 2, dadurch gekennzeichnet, daß man von einem Gemisch aus IIa und IIb ausgeht, wie es bei der Umsetzung eines Ketoxims VII 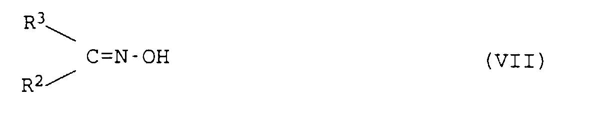 mit einem Alkylenoxid VIII 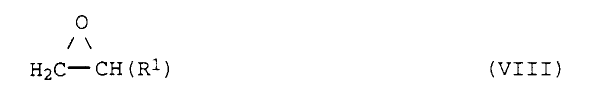 oder dem entsprechenden Carbonat IX 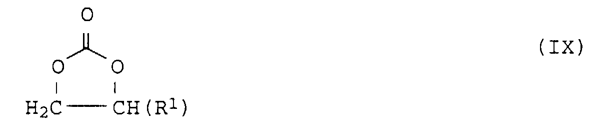 anfällt.

7. Verfahren nach Anspruch 1 oder 3, dadurch gekennzeichnet, daß man die Hydroylse der Mischung aus VIa und VIb mit Salzsäure, Schwefelsäure oder Phosphorsäure vornimmt.

8. verfahren nach den Ansprüchen 1 bis 3, dadurch gekennzeichnet, daß R¹, R² und R³ in den Ausgangsmischungen jeweils Methyl bedeuten.

9. Verfahren zur Herstellung von Isomerengemischen, bestehend aus den Hydrochlorid- oder Sulfat-Salzen von Ia und Ib gemäß Anspruch 1 oder 3, dadurch gekennzeichnet, daß man eine Mischung aus O-Phenoxyalkyloximen VIa und VIb, wobei R² und R³ für Methyl stehen, mit wäßriger Salzsäure oder Schwefelsäure hydrolysiert und das hierbei anfallende Aceton laufend aus dem Reaktionsgemisch entfernt.

10. Isomerengemisch aus {O-[2-(4-Chlorphenoxy)-propyl]-hydroxylammonium}-hydrogensulfat 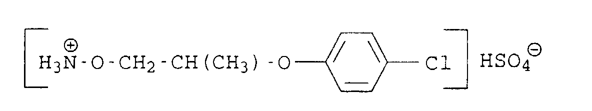 und {O-[2-(4-Chlorphenoxy)-1-methylethyl]-hydroxylammonium}-hydrogensulfat

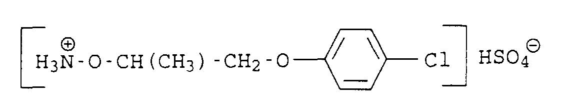

11. Isomerengemisch aus O-[2-(4-Chlorphenoxy)-propy]-hydroxylammoniumchlorid 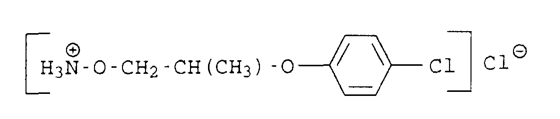 und O-[2-(4-Chlorphenoxy)-1-methylethyl]-hydroxylammoniumchlorid

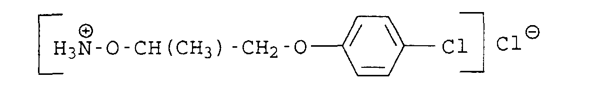

12. Isomerengemisch aus O-[2-Methylsulfonyloxypropyl]-propan-2-onoxim 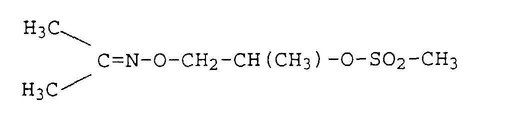 und O-[2-Methylsulfonyloxy-1-methylethyl]-propan-2-onoxim

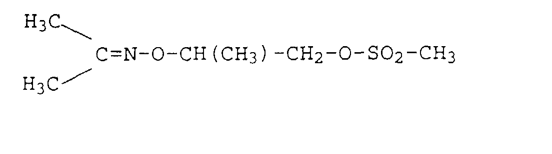

## Claims

1. A process for preparing mixtures of isomers of O-phenoxyalkylhydroxylamines of the general formulae Ia and Ib
H₂N-O-CH₂-CH(R¹)-O-Ar (Ia)
H₂N-O-CH(R¹)-CH₂-O-Ar (Ib)
where R¹ is alkyl and Ar is phenyl which can carry nonaromatic substituents and the corresponding salt mixtures, which comprises
a) converting a mixture of isomers of O-(2-hydroxyethyl)oximes of the formulae IIa and IIb 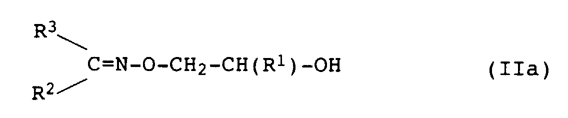 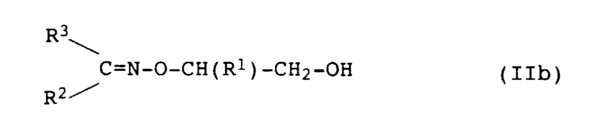 where R² is alkyl and R³ is alkyl or alkoxy, or R² and R³ form, together with the common carbon atom, a 5- to 7-membered isocyclic ring,
with a sulfonyl halide of the formula III
Hal-SO₂-R⁴ (III)
where R⁴ is C₁-C₄-alkyl, C₁-C₄-haloalkyl or
C₁-C₄-alkylphenyl, and Hal is halogen, in the presence of a base into the corresponding mixture of sulfonates IVa and IVb 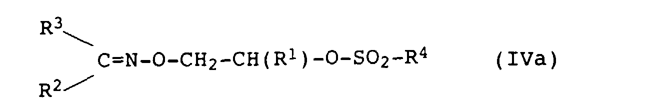 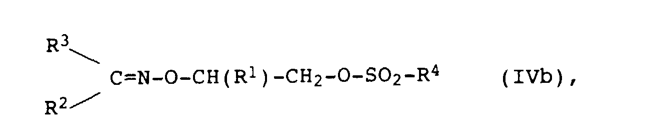
b) reacting this mixture of sulfonates in the presence of a base with a phenol of the formula V
HO-Ar (V)
to give a mixture of O-phenoxyalkyloximes of the general formulae VIa and VIb 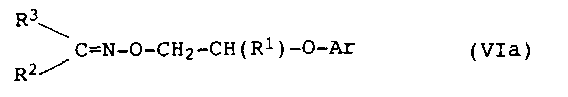 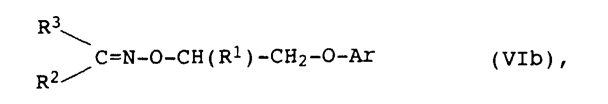
c) hydrolyzing this mixture in the presence of an acid and, if required,
d) liberating the O-phenoxyalkylhydroxylamines Ia and Ib from the resulting salts using a mineral base.

2. A process for preparing mixtures of O-phenoxyalkyloximes of the general formulae VIa and VIb 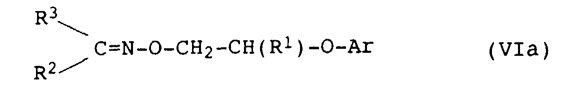 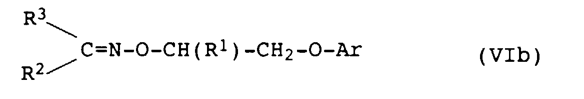 where R¹ to R³ and Ar have the meanings given in claim 1, which comprises
a) converting a mixture of isomers of O-(2-hydroxyethyl)oximes of the formulae IIa and IIb 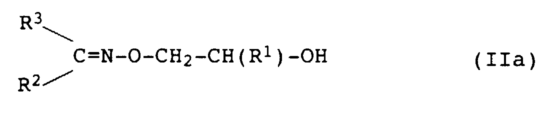 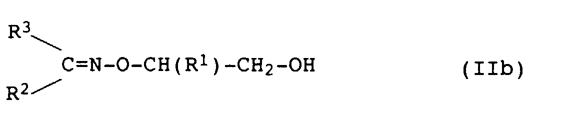 with a sulfonyl halide of the formula III
Hal-SO₂-R⁴ (III)
where R⁴ is C₁-C₄-alkyl, C₁-C₄-haloalkyl or
C₁-C₄-alkylphenyl, and Hal is halogen, in the presence of a base into the corresponding mixture of sulfonates IVa and IVb 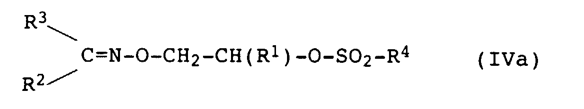 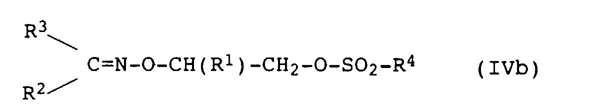 and
b) reacting this mixture of sulfonates in the presence of a base with a phenol of the formula V
HO-Ar (V).

3. A process for preparing mixtures of isomers of O-phenoxyalkylhydroxylamines of the formulae Ia and Ib
H₂N-O-CH₂-CH(R¹)-O-Ar (Ia)
H₂N-O-CH(R¹)-CH₂-O-Ar (Ib)
where R¹ and Ar have the meanings given in claim 1, and the corresponding salt mixtures, which comprises hydrolyzing a mixture of the O-phenoxyalkyloximes VIa and VIb 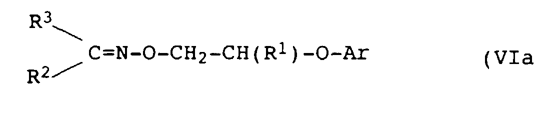 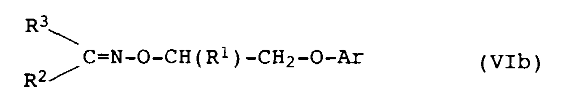 where R² is alkyl and R³ is alkyl or alkoxy, or R² and R³ form, together with the common carbon atom, a 5- to 7-membered isocyclic ring,
in the presence of an acid, and if required reacting the salts of Ia and Ib produced thereby with a base.

4. A process as claimed in claim 1 or 2, wherein an aliphatic tertiary amine is employed as base in process stage (a), and a mineral base is employed in process stage (b).

5. A process as claimed in claim 1 or 2, wherein the mixture of sulfonates IVa and IVb is, without isolating it, reacted with the phenol V to give a mixture of O-phenoxyalkyloximes Ia and Ib.

6. A process as claimed in claim 1 or 2, which starts from a mixture of IIa and IIb as produced in the reaction of a ketoxime VII 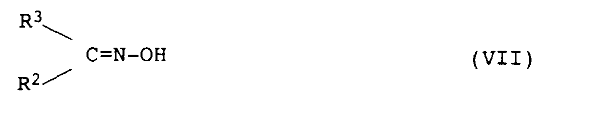 with an alkylene oxide VIII 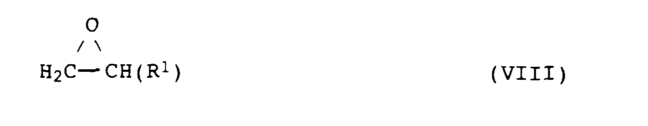 or the corresponding carbonate IX

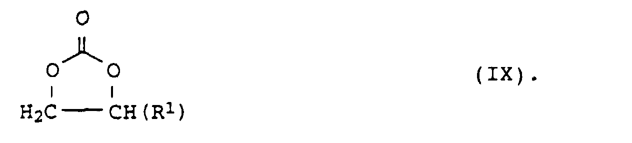

7. A process as claimed in claim 1 or 3, wherein the hydrolysis of the mixture of VIa and VIb is carried out with hydrochloric acid, sulfuric acid or phosphoric acid.

8. A process as claimed in any of claims 1 to 3, wherein R¹, R² and R³ in the starting mixtures are each methyl.

9. A process for preparing mixtures of isomers comprising the hydrochloride or sulfate salts of Ia and Ib as claimed in claim 1 or 3, which comprises hydrolyzing a mixture of O-phenoxyalkyloximes VIa and VIb, where R² and R³ are methyl, with aqueous hydrochloric acid or sulfuric acid, and continuously removing the acetone produced thereby from the reaction mixture.

10. A mixture of the isomers O-[2-(4-chlorophenoxy)propyl]hydroxylammonium bisulfate 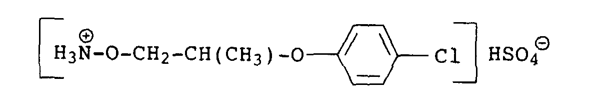 and O-[2-(4-chlorophenoxy)-1-methylethyl]hydroxylammonium bisulfate

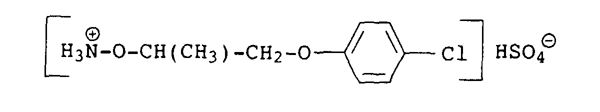

11. A mixture of the isomers O-[2-(4-chlorophenoxy)propyl]hydroxylammonium chloride 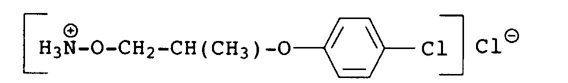 and O-[2-(4-chlorophenoxy)-1-methylethyl]hydroxylammonium chloride

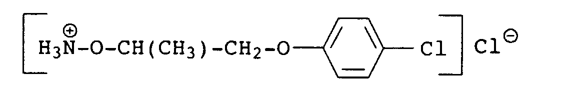

12. A mixture of the isomers 2-propanone O-[2-methylsulfonyloxypropyl]oxime 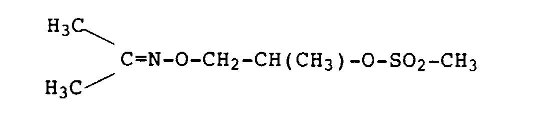 and 2-propanone O-[2-methylsulfonyloxy-1-methylethyl]oxime

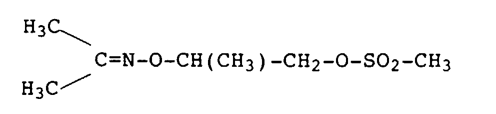

## Revendications

1. Procédé pour la préparation de mélanges d'isomères d'O-phénoxyalkylhydroxylamines de formules générales Ia et Ib
H₂N-O-CH₂-CH(R¹)-O-Ar (Ia)
H₂N-O-CH(R¹)-CH₂-O-Ar (Ib)
où R¹ désigne un groupe alkyle et Ar désigne le groupe phényle, qui peut porter des substituants non aromatiques, ainsi que les mélanges de sels correspondants, caractérisé par le fait qu'on
a) convertit un mélange d'isomères d'O-(2-hydroxyéthyl)-oximes de formules IIa et IIb 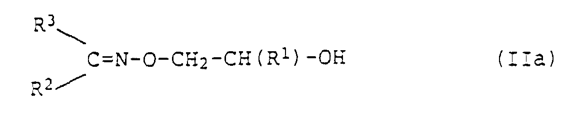 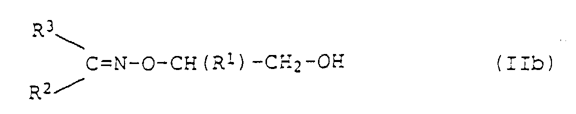 où R² désigne un groupe alkyle et R³ représente un groupe alkyle ou alcoxy, ou R² et R³ forment ensemble et avec l'atome de carbone commun un cycle isocyclique à 5 à 7 chaînons,
avec un halogénure de sulfonyle de formule III
Hal-SO₂-R⁴ (III)
où R⁴ désigne un groupe alkyle en C₁-C₄, halogénoalkyle en C₁-C₄ ou alkylphényle en C₁-C₄ et Hal représente un halogène, en présence d'une base, en le mélange de sulfonates correspondants IVa et IVb 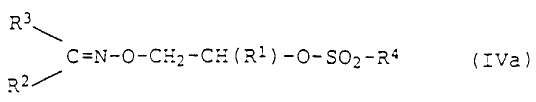 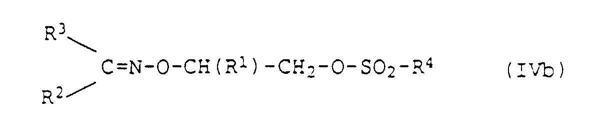
b) fait réagir ce mélange de sulfonates, en présence d'une base, avec un phénol de formule V
HO-Ar (V)
pour former un mélange d'O-phénoxyalkyloximes de formules générales VIa et VIb 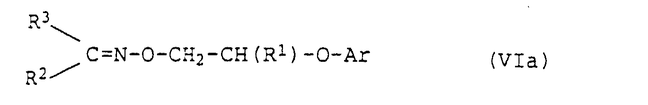 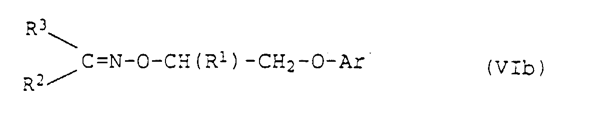
c) hydrolyse ce mélange en présence d'un acide et éventuellement,
d) libère à partir des sels qui se forment alors les O-phénoxyalkylhydroxylamines Ia et Ib au moyen d'une base minérale.

2. Procédé pour la préparation de mélanges d'O-phénoxyalkyloximes de formules générales VIa et VIb 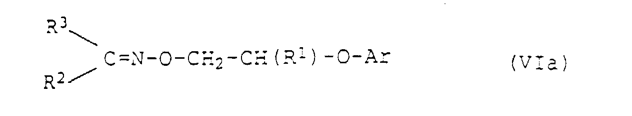 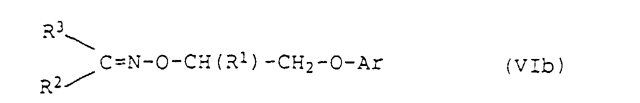 où R¹ à R³ et Ar ont les significations indiquées dans la revendication 1, caractérisé par le fait qu'on
a) convertit un mélange d'isomères d'O-(2-hydroxyéthyl)-oximes de formules IIa et IIb 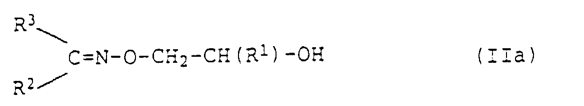 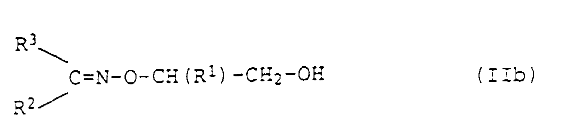 avec un halogénure de sulfonyle de formule générale III
Hal-SO₂-R⁴ (III)
où R⁴ désigne un groupe alkyle en C₁-C₄, halogénoalkyle en C₁-C₄ ou alkylphényle en C₁-C₄ et Hal représente un halogène, en présence d'une base, en le mélange de sulfonates correspondants IVa et IVb 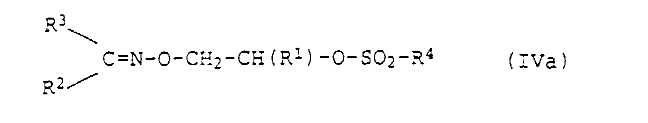 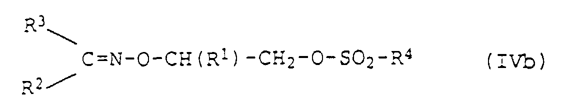
b) fait réagir ce mélange de sulfonates, en présence d'une base, avec un phénol de formule V
HO-Ar (V)

3. Procédé pour la préparation de mélanges d'isomères d'O-phénoxyalkylhydroxylamines de formules Ia et Ib
H₂N-O-CH₂-CH(R¹)-O-Ar (Ia)
H₂N-O-CH(R¹)-CH₂-O-Ar (Ib)
où R¹ et Ar ont les significations indiquées dans la revendication 1, ainsi que les mélanges de sels correspondants, caractérisé par le fait qu'on hydrolyse un mélange d'O-phénoxyalkyloximes VIa et IVb 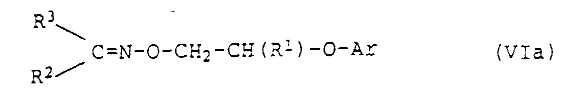 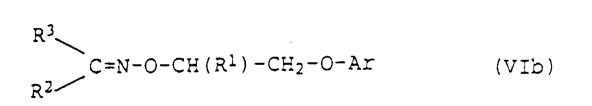 où R² représente un groupe alkyle et R³ désigne un groupe alkyle ou alcoxy, ou R² et R³ forment ensemble et conjointement avec l'atome de carbone commun un cycle isocyclique à 5 à 7 chaînons,
en présence d'un acide et on fait éventuellement réagir avec une base les sels ainsi formés de Ia et Ib.

4. Procédé selon la revendication 1 ou 2, caractérisé par le fait qu'on utilise comme base dans l'étape opératoire (a) une amine tertiaire aliphatique et dans l'étape opératoire (b), une base minérale.

5. Procédé selon la revendication 1 ou 2, caractérisé par le fait qu'on fait réagir le mélange de sulfonates de IVa et IVb, sans l'isoler, avec le phénol V pour donner un mélange d'O-phénoxyalkyloximes Ia et Ib.

6. Procédé selon la revendication 1 ou 2, caractérisé par le fait qu'on part d'un mélange de IIa et IIb, tel qu'il se forme dans la réaction d'une cétoxime VII 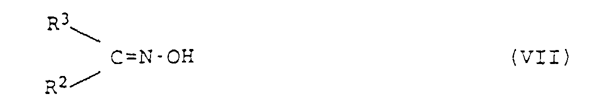 avec un oxyde d'alkylène VIII 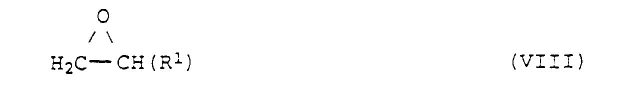 ou avec le carbonate correspondant IX

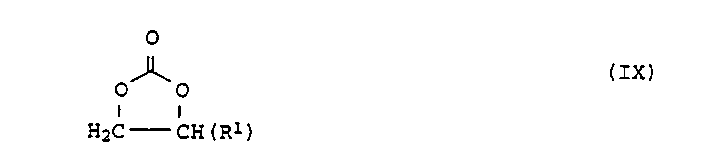

7. Procédé selon la revendication 1 ou 3, caractérisé par le fait qu'on effectue l'hydrolyse du mélange de VIa et VIb avec de l'acide chlorhydrique, de l'acide sulfurique ou de l'acide phosphorique.

8. Procédé selon les revendications 1 à 3, caractérisé par le fait que R¹, R² et R³ dans les mélanges de départ représentent chacun un groupe méthyle.

9. Procédé pour la préparation de mélanges d'isomères, consistant en les sels de chlorhydrate ou de sulfate de Ia et Ib selon la revendication 1 ou 3, caractérisé par le fait qu'on hydrolyse un mélange d'O-phénoxyalkyloximes VIa et VIb, où R² et R³ désignent un groupe méthyle, avec de l'acide chlorhydrique ou de l'acide sulfurique aqueux et on élimine au fur et à mesure l'acétone qui se forme alors à partir du mélange réactionnel.

10. Mélanges d'isomères d'hydrogénosulfate de {O-[2-(4-chlorophénoxy)-propyl]-hydroxylammonium} 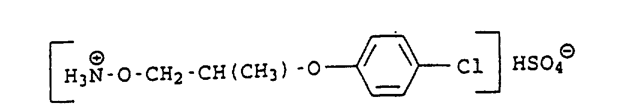 et d'hydrogénosulfate de {O-[2-(4-chlorophénoxy)-1-méthyléthyl]-hydroxylammonium}

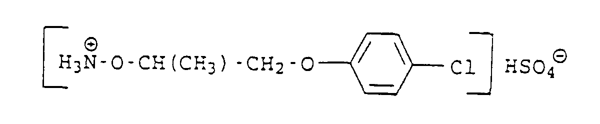

11. Mélanges d'isomères de chlorure d'O-[2-(4-chlorophénoxy)-propyl]-hydroxylammonium} 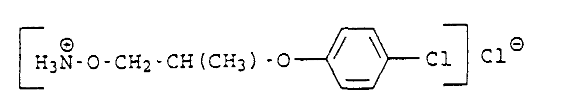 et de chlorure d'O-[2-(4-chlorophénoxy)-méthyléthyl]hydroxylammonium}

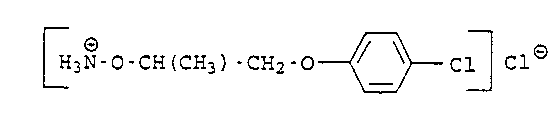

12. Mélanges d'isomères d'O-[2-méthylsulfonyloxypropyl]propane-2-onoxime 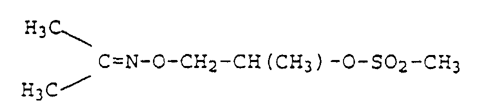 et d'O-[2-méthylsulfonyloxy-1-méthyléthyl]-propane-2-onoxime
